# EUROPÄISCHE PATENTSCHRIFT

(11) **EP 1 161 863 B1**
(45) Veröffentlichungstag und Bekanntmachung des Hinweises auf die Patenterteilung: **10.12.2003**
(21) Anmeldenummer: 01111092.1
(22) Anmeldetag: 09.05.2001
(51) Int. Cl.: A01K 67/033

(54) **Behältnis zum Ausbringen von Nützlingen**
Container for spreading useful organisms
Conteneur pour épandre des organismes utiles

(30) Priorität: 07.06.2000 DE 10027767
(43) Veröffentlichungstag der Anmeldung: 12.12.2001
(73) Patentinhaber: fischerwerke Artur Fischer GmbH & Co. KG, 72178 Waldachtal (DE); AMW Nützlinge GmbH, 64319 Pfungstadt (DE)
(72) Erfinder: Herbstreit, Rolf, 72178 Waldachtal (DE); Wührer, Bernd, Dr., 64319 Pfungstadt (DE)

(56) Entgegenhaltungen:
- US-A- 4 785 764

## Beschreibung

Die Erfindung betrifft ein Behältnis zum Ausbringen von Nützlingen insbesondere Schlupfwespen.

Schlupfwespen (Trichogramma) werden für die biologische Schädlingsbekämpfung eingesetzt. Die Eiparasiten der Schlupfwespen sind die natürlichen Feinde vieler Schädlinge im Acker-, Gemüse- und Obstanbau. Die Schlupfwespen legen ihre eigenen Eier in die Eier der Schädlinge. Sie töten diese ab und entwickeln sich darin zum flugreifen Vollinsekt. Erwachsene Schlupfwespen ernähren sich ausschließlich von Pollen und Honigtau. Natürlich vorkommende Populationen reichen für eine erfolgreiche Bekämpfung nicht aus. Schlupfwespen werden deshalb in Massen vermehrt und ausgebracht.

Zum Ausbringen werden die Schlupfwespen-Eier auf einem Kartonträger (TrichoKarte) aufgeklebt, der an die zu schützende Pflanze angehängt wird. Bei großflächiger Schädlingsbekämpfung beispielsweise für Maisfelder sind eine Vielzahl von TrichoKarten auszubringen, was durch entsprechenden Personaleinsatz von Hand erfolgt.

Weiterhin ist aus der Druckschrift US-A-4785764 ein Behältnis zum Ausbringen von Nützlingen vorgeschlagen, das im Wesentlichen aus einem Hohlkörper mit einer im unteren Bereich des Hohlkörpers angeordneten Abdeckung besteht, wobei die Abdeckung Ausschlupföffnungen in Form eines Siebgewebes oder einzelne Öffnungen aufweist. In den Hohlkörper werden Nützlinge eingebracht, die darin geschützt sind und von denen nur solche einer gewünschten Größe das Siebgewebe passieren können. Das Behältnis ist aus einem Material, das durch eine chemische oder physikalische Behandlung eine eingestellte Intaktheitsdauer aufweist und zur schnellen Verwitterung nach dem Schlüpfen der Nützlinge bestimmt ist. Das Problem eines solchen Behältnisses besteht darin, dass es ungeeignet ist, um eine magazinierte Ausbringung beispielsweise mit einem Kleinflugzeug zu realisieren. So schlägt die Druckschrift US-A-4785764 vor, das die Ausschlupföffnungen einen rechteckigen oder quadratischen Querschnitt aufweisen, wobei das Längen-Breitenverhältnis bei maximal 2 liegt. Die Abmessungen orientieren sich dabei an den frisch geschlüpften Nützlingen. Sie sind gerade so groß, dass die Nützlinge entweichen können, aber auch nicht wesentlich größer, um ihnen einen bestmöglichen Schutz zu bieten. Gerade quadratische Querschnitte neigen bei derartigen Dimensionen jedoch dazu, aufgrund der Oberflächenspannung von Wasser bei einmaligem Kontakt zu benetzen. Hierdurch entsteht ein hartnäckiger Verschluss der Ausschlupföffnungen, der beispielsweise eine Belüftung des Behältnisses verhindert. Eine solche Belüftung ist jedoch für das Wachstum der sich entwickelnden Insekten von großer Bedeutung. Des Weiteren verursacht ein vollständiges Benetzen der Öffnungen, dass einmal eingedrungenes Wasser auch nur schlecht wieder abfließen kann, da es einerseits durch die Oberflächenspannung zurückgehalten wird und andererseits kein Druckausgleich möglich ist. Auch dies verhindert die Zufuhr von Luft zu den schlüpfenden Nützlingen. Aufgrund dieser Probleme sind die aus der Druckschrift US-A-4785764 bekannten Behältnisse grundsätzlich so auszubringen, dass die Ausschlupföffnungen nach unten weisen und im Abstand zum umgebenden Boden angeordnet sind. Hierdurch sind sie gegen Regenwasser und Schmutz geschützt, allerdings ist es erforderlich, die Behältnisse entweder an einem Bügel aufzuhängen oder mit einem Pfahl oder dgl. aufzuständern. Dies erfordert einen sehr aufwändigen manuellen Prozess zum Ausbringen der Behältnisse.

Der Erfindung liegt die Aufgabe zu Grunde, ein stabiles und rückstandsfrei sich zersetzendes Behältnis zum Ausbringen von Nützlingen zu schaffen, das eine automatische Befüllung und eine magazinierte Ausbringung beispielsweise mit Kleinflugzeugen ermöglicht.

Die Lösung dieser Aufgabe wird erfindungsgemäß durch die Merkmale des Anspruchs 1 erreicht. Die beiden vorzugsweise als Halbkugeln und aus biologisch abbaubarem Kunststoff (BAK) hergestellten Teile des Behältnisses werden getrennt in einer automatischen Befüllungsanlage mit jeweils ca. 1000 Schlupfwespen-Eiern gefüllt und zusätzlich an die Innenwandung der Halbkugeln geklebt. Danach werden die beiden Halbkugeln aufeinander gesetzt und beispielweise über eine Clipsverbindung miteinander verbunden. Durch die Herstellung des Behältnisses im Spritzgussverfahren aus einem biologisch abbäubaren Kunststoff und der Schalen- insbesondere Kugelform des Behältnisses ergibt sich eine Steifigkeit, die es zulässt, das Behältnis sowohl in ein Magazin einzubringen als auch aus einer größeren Höhe - beispielsweise bei der Ausbringung mit einem Klein- oder Modellflugzeug - abzuwerfen, ohne dass das Behältnis zerstört wird.

Wenige Tage nach dem Ausbringen schlüpfen die Schlupfwespen und verlassen über die Ausschlupföffnungen das Behältnis. Durch ihre Eiablage in den Eiern der Schädlinge werden diese abgetötet. Aus den Eiern der Schlupfwespe entschlüpft dann die nächste Generation und setzt den Populationsprozess zum Schutz der Pflanzen fort. Das leere aus biologisch abbaubarem Kunststoff bestehende Behältnis zersetzt sich insbesondere nach dem Unterpflügen rückstandsfrei im Boden.

Die Erfindung sieht vor, die Ausschlupföffnungen schlitzförmig auszubilden, wobei die Querschnittsfläche der Ausschlupföffnung etwa 0,5 mm in der Breite und 5 mm in der Länge bemisst. Die Schlitzförmigkeit bewirkt einerseits, dass eine ausreichend große Öffnung für das Ausschlüpfen gegeben ist. Hierfür ist die Breite das bestimmende Maß, welches entsprechend der Größe der frisch geschlüpften Schlupfwespen im Bereich von 0,5 mm liegt. Andererseits bewirkt die Schlitzform, dass die Ausschlupföffnungen durch Einwirkung von Regenwasser und den Kontakt zum Erdreich nicht vollständig verschließen. Daneben ergibt sich als Vorteil der Schlitzförmigkeit, dass diese bei der üblichen spritztechnischen Realisierung deutlich einfacher herstellbar ist als quadratische oder runde Öffnungen.

Zum kippfreien Aufsetzen der Halbkugeln auf einem Werkstückträger für die automatische Befüllung ist es zweckmäßig, die Halbkugeln an ihrem äußeren Rand mit einem umlaufenden Bund zu versehen.

Schließlich kann in weiterer Ausgestaltung das Behältnis mit einer Aufhängung versehen sein, die auch die Einzelaufhängung der Behältnisse an Sträuchern oder Bäumen ermöglicht.

Die Erfindung wird nachfolgend anhand in der Zeichnung dargestellter Ausführungsbeispiele näher erläutert. Es zeigen:
- Figur 1: das aus zwei Teilen bestehende Behältnis in geöffnetem und
- Figur 2: in geschlossenem Zustand

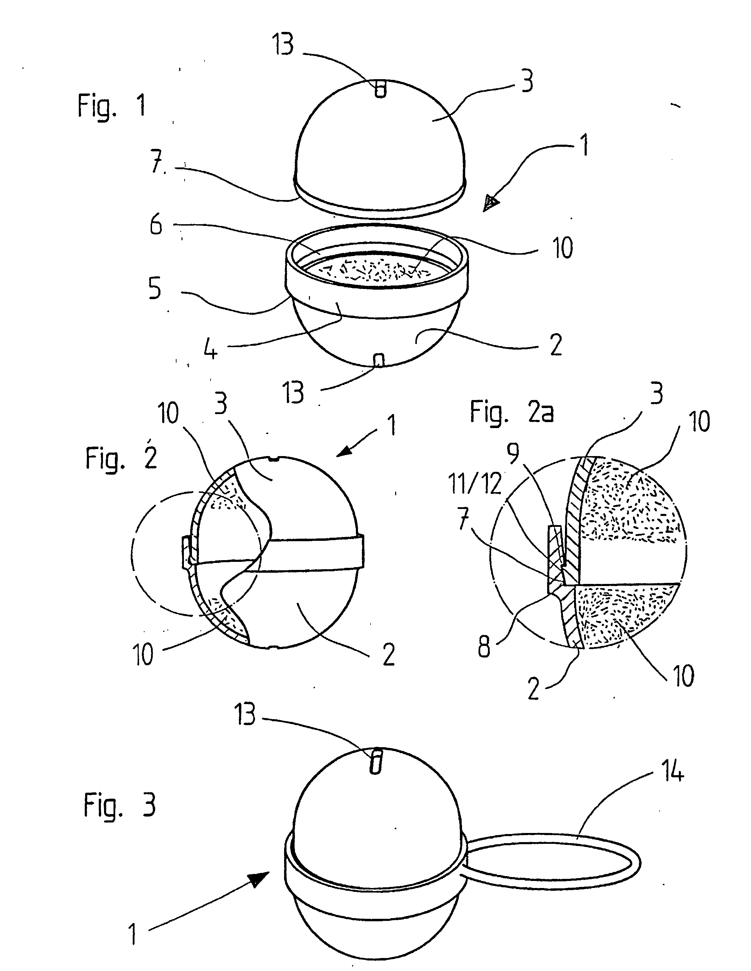

- Figur 2 a: eine Detaildarstellung der Rastmittel und
- Figur 3: das Behältnis mit einer Aufhängung.

Das Behältnis 1 besteht aus zwei schalenförmigen, als Halbkugeln ausgebildeten Teilen 2, 3, die im Spritzgießverfahren aus biologisch abbaubarem Kunststoff (BAK) hergestellt sind. Im Zenitpunkt der beiden Teile 2, 3 ist jeweils eine schlitzförmige Ausschlupföffnung 13 vorgesehen, die etwa die Maße 0,5 x 5 mm aufweist. Das eine Teil 2 weist an seinem äußeren Rand einen umlaufenden Bund 4 auf, der sowohl an der Innen- als auch an der Außenwandung des Teils eine Auflagefläche 5, 6 bildet. Mit der äußeren Auflagefläche 5 kann das Teil 2 in eine Aufnahme eines Werkstückträgers einer Vorrichtung kippfrei eingesetzt und zur Befüllungsstation einer Vorrichtung befördert werden (nicht dargestellt). Die innere Anschlagfläche 6 dient zum Aufsetzen des anderen Teils 3, das ebenfalls einen umlaufenden Bund 7 aufweist, der in die Innenbohrung des Bunds 4 des Teils 2 passt. Um das Einstecken des Teils 3 in das Teil 2 zu erleichtern, weist der Bund 7 des Teils 3 eine Anlaufschräge 8 auf. Der Bund 7 des Teils 3 bildet ebenfalls eine äußere Auflagefläche 9, die das kippfreie Aufsetzen des Teils 3 in die Aufnahme eines Werkstückträgers ermöglicht.

Nach dem Befüllen der Teile 2, 3 mit den Schlupfwespeneiern 10 und deren Verkleben mit der Innenwandung der Teile werden die beiden Teile 2, 3 zusammengesteckt und durch Verkleben, Verklemmen oder wie im Ausführungsbeispiel dargestellt, durch Clipsen miteinander verbunden. Als Rastmittel weist das Teil 2 an der Innenwandung des Bundes 4 eine umlaufende Rille 11 und das Teil 3 am Bund 7 angeordnete Vorsprünge 12 auf.

Nach dem Einrasten ergibt sich ein kugelförmiges, geschlossenes Behältnis 1, das eine hohe Steifigkeit für die Aufnahme in einem Magazin und für den zerstörungsfreien Abwurf für eine großflächige Ausbringung in einem Klein- oder Modelflugzeug ermöglicht. Die magazinierten Einzelbehältnisse dienen vorwiegend zur biologischen Schädlingsbekämpfung von größeren Flächen, beispielsweise Maisfeldern. Für die Schädlingsbekämpfung bei Bäumen, Sträuchern oder dergleichen kann gemäß dem Ausführungsbeispiel nach Figur 3 das Behältnis 1 mit einer Aufhängung 14 versehen sein, die eine Einzelaufhängung eines Behältnisses ermöglicht. Für eine intensivere Schädlingsbekämpfung können mehrere Behältnisse beispielsweise wabenförmig miteinander verbunden werden.

## Patentansprüche

1. Behältnis zum Ausbringen von Nützlingen insbesondere Schlupfwespen, wobei das Behältnis (1) aus zwei miteinander verbindbaren Teilen (2, 3) aus einem biologisch abbaubaren Material besteht und wenigstens eine Ausschlupföffnung (13) aufweist, **dadurch gekennzeichnet, dass** die Ausschlupföffnung (13) schlitzförmig ausgebildet ist, wobei die Querschnittsfläche der Ausschlupföffnung etwa 0,5 mm in der Breite und 5 mm in der Länge bemisst.

2. Behältnis nach Anspruch 1, **dadurch gekennzeichnet, dass** die beiden Teile (2, 3) schalenförmig vorzugsweise als Halbkugeln ausgebildet sind und an ihrem äußeren Rand jeweils einen umlaufenden Bund (4, 7) aufweisen, die zur Bildung eines geschlossenen Behältnisses ineinander steckbar und verrastbar sind.

## Claims

1. Container for the delivery of useful organisms, especially ichneumon flies, wherein the container (1) consists of two parts (2, 3), made from a biodegradable material, that are connectible to one another and has at least one emergence opening (13), **characterized in that** the emergence opening (13) is slit-shaped, the cross-sectional surface area of the emergence opening measuring approximately 0.5 mm in width and 5 mm in length.

2. Container according to claim 1, **characterized in that** the two parts (2, 3) are dish-shaped, preferably hemispherical, and each of the two dish-shaped parts (2, 3) has a respective circumferential collar (4, 7) at its outer rim, which collars can be inserted and locked one into the other to form a closed container.

## Revendications

1. Conteneur pour épandre des organismes utiles, en particulier des trichogrammes, le conteneur (1) étant constitué de deux parties (2, 3) combinables entre elles, fabriqué à partir d'une matière biologiquement dégradable, et présentant au moins une ouverture d'éclosion (13), **caractérisé en ce que** l'ouverture d'éclosion (13) est réalisée en forme de fente, la section transversale de l'ouverture d'éclosion ayant pour dimension environ 0,5 mm en largeur et 5 mm en longueur.

2. Conteneur selon la revendication 1, **caractérisé en ce que** les deux parties (2, 3) sont réalisées de préférence en forme d'hémisphères, et que sur leur bord extérieur, elles présentent chaque fois un collet circulaire (4, 7), et qu'elles sont embrochables et encliquetables l'une dans l'autre pour former un conteneur fermé.
